# EUROPÄISCHE PATENTSCHRIFT

(11) **EP 1 816 217 B1**
(45) Veröffentlichungstag und Bekanntmachung des Hinweises auf die Patenterteilung: **15.06.2011**
(21) Anmeldenummer: 07100321.4
(22) Anmeldetag: 10.01.2007
(51) Int. Cl.: C12Q 1/68

(54) **Verfahren zum Nachweis mehrerer Zielnukleinsäuren**
Method for the detection of a plurality of target nucleic acids
Méthode pour la détection d'une pluralité d'acides nucléiques cibles

(30) Priorität: 06.02.2006 DE 102006005287
(43) Veröffentlichungstag der Anmeldung: 08.08.2007
(73) Patentinhaber: Siemens Aktiengesellschaft, 80333 Munich (DE)
(72) Erfinder: Gumbrecht, Walter, Dr., 91074 Herzogenaurach (DE); Mosner, Jörn, Dr., 91054 Erlangen (DE); Stanzel, Manfred, Dr., 91056 Erlangen (DE); Zilch, Christian, Dr., 04275 Leipzig (DE)

(56) Entgegenhaltungen:
- WO-A-2007/000408
- US-A1- 2001 018 412
- US-A1- 2002 119 470
- US-A1- 2004 096 857
- STRIZHKOV BORIS N ET AL: "PCR amplification on a microarray of gel-immobilized oligonucleotides: Detection of bacterial toxin- and drug-resistant genes and their mutations" BIOTECHNIQUES, INFORMA LIFE SCIENCES PUBLISHING, WESTBOROUGH, MA, US, Bd. 29, Nr. 4, Oktober 2000 (2000-10), Seiten 844-857, XP002167651 ISSN: 0736-6205
- DRESSMAN DEVIN ET AL: "Transforming single DNA molecules into fluorescent magnetic particles for detection and enumeration of genetic variations." PROCEEDINGS OF THE NATIONAL ACADEMY OF SCIENCES OF THE UNITED STATES OF AMERICA 22 JUL 2003, Bd. 100, Nr. 15, 22. Juli 2003 (2003-07-22), Seiten 8817-8822, XP002434941 ISSN: 0027-8424
- ANDREADIS J D ET AL: "Use of immobilized PCR primers to generate covalently immobilized DNAs for in vitro transcription/translation reactions." NUCLEIC ACIDS RESEARCH 15 JAN 2000, Bd. 28, Nr. 2, 15. Januar 2000 (2000-01-15), Seite e5, XP002434942 ISSN: 1362-4962

## Beschreibung

Die Erfindung betrifft ein Verfahren zum Nachweis mehrerer Zielnukleinsäuren unter Verwendung von an einen Semi-Festphasen-Träger gekoppelten Primern.

Nukleinsäure-Amplifikationsverfahren sind im Stand der Technik bekannt. Am häufigsten angewandt wird die Polymerase-Ketten-Reaktion (PCR). Dieses Verfahren ermöglicht die Vervielfältigung von Nukleinsäuremolekülen und basiert auf der Replikation von Nukleinsäuren mit Hilfe von hitzebeständigen Polymerasen. Hierbei wird ein Paar von Oligonukleotid-Primern (einzelsträngige Oligonukleotide) mit der zu amplifizierenden Nukleinsäure in Kontakt gebracht. Die Primer werden so gewählt, dass sie an den beiden Enden eines zu amplifizierenden Fragments auf den komplementären Strängen binden. Bei der Elongation wird dann der eine Primer in einer Richtung und der andere Primer in der entgegengesetzten Richtung entlang der Zielnukleinsäure in 3'-Richtung elongiert (Vorwärts- und Rückwärts-Primer). Vorwärts- und Rückwärts-Primer werden alternativ auch als Sense- oder Antisense-Primer bezeichnet. Auf diese Weise kann das zwischen den zu den Primern komplementären Stellen auf der Zielnukleinsäure liegende Stück amplifiziert werden. Für nachfolgende Nachweisreaktionen werden die PCR Produkte vorteilhafterweise von Primern, Nukleotiden und anderen störenden Komponenten des PCR-Ansatzes abgetrennt.

Der PCR-Ablauf umfasst mehrere Thermozyklen mit jeweils drei Schritten: Zunächst wird die in der Probe enthaltene doppelsträngige DNA erhitzt, um die Stränge zu trennen (Denaturierung). Daraufhin wird die Temperatur gesenkt, so dass die Primer sich an die DNA-Einzelstränge anlagern können (Annealing). Im letzten Schritt wird der DNA-Abschnitt zwischen den Primern durch die Polymerase mit den jeweils komplementären Nukleotiden aufgefüllt (Elongation). Dieser Zyklus wird typischerweise ca. 15 - 50 mal durchlaufen.

Biochips messen die Konzentration bzw. das Vorhandensein von Biomolekülen (z.B. DNA, Proteine) in biologischen Proben. Bei DNA-Mikroarrays befinden sich spezifische Fängermoleküle auf distinkten Stellen (Spots) auf der Oberfläche geeigneter Träger gekoppelt (wie z.B. Glas, Plexiglas, Silizium). Bei den Fängermolekülen handelt es sich in der Regel um einzelsträngige Oligonukleotide (15 - 40 Basenpaare) oder alternativ einzelsträngige PCR-Produkte, die gegen spezifische Zielmoleküle in einer zu untersuchenden Probe gerichtet sind. Die einzelsträngigen Fängermoleküle hybridisieren bei definierter Stringenz (Temperatur, Pufferbedingungen) mit entsprechenden komplementären einzelsträngigen Zielmolekülen (beispielsweise aus einem PCR-Produkt) und können mit Hilfe verschiedener Nachweisverfahren identifiziert werden. Zu diesem Zweck kommen meist optische, elektrische oder magnetische Detektionsverfahren zum Einsatz.

Allen Detektionsverfahren gemein ist die Voraussetzung, dass nur einzelsträngige Nukleinsäuren in der Lage sind, von den am Mikroarray befindlichen Fängermolekülen gebunden zu werden (mit Ausnahme der exotischen 3-strängigen Hybride). Diese Voraussetzung ist bei der Verwendung von RNA-Proben bereits erfüllt. Sollen spezifische DNA-Sequenzen beispielsweise bei Genotypisierungen und der Mutationsdetektion (SNP-Analyse), aber auch cDNA in Expression-Profile-Experimenten nachgewiesen werden, muss vorher die in Form eines Doppelstrangs vorliegende DNA in seine Einzelstränge aufgespalten (denaturiert) werden. Ein typisches PCR-Produkt, cDNA oder durch Restriktionsenzyme erzeugtes doppelsträngiges DNA-Fragment wird in aller Regel durch Erhitzen auf ca. 95°C in seine Einzelstränge aufgespalten. Alternativ bzw. unterstützend zur thermischen Aufspaltung können stark alkalische Agenzien wie z.B. Natronlauge zur Strangtrennung verwendet werden.

Als problematisch hat sich hierbei erwiesen, dass sich die voneinander getrennten Einzelstränge besonders bei niedriger Stringenz wieder zusammenlagern können. Insbesondere während des Hybridisierungsvorgangs auf einem Mikroarray tritt der Prozess des sog. Reannealings in Konkurrenz mit der Hybridisierung an den jeweils spezifischen Fängermolekülen auf der Oberfläche des Mikroarrays. Dieser Vorgang wirkt sich unvorteilhaft auf die Sensitivität des Assays aus, da die Hybridisierung gesuchter Nukleinsäuresequenzen durch Reannealing signifikant verringert werden kann. Insbesondere bei einer geringen Konzentration reicht die Empfindlichkeit oftmals nicht aus, um die Zielsequenzen nachzuweisen.

Ein weiteres Problem bei Mikroarray-Experimenten mit vorgelagerter PCR-Reaktion ist die Tatsache, dass die Amplifikation auf eine geringe Anzahl von Produktspezien beschränkt ist. Dieser Umstand erweist sich insbesondere dann als besonders problematisch, wenn sich die Schmelztemperaturen der unterschiedlichen PCR-Primer-Paare oder die Länge der resultierenden PCR-Produkte signifikant unterscheiden.

In diesen Fällen werden bei einer sog. Multiplex-PCR einige Sequenzen bevorzugt amplifiziert, so dass nach einigen wenigen PCR-Zyklen ein Ungleichgewicht der Konzentrationen von PCR-Produkten resultiert. Die durch die PCR entstehenden ungleichen Produktverhältnisse erweisen sich jedoch bei einem nachfolgenden Nachweis der Zielmoleküle (PCR-Produkte) auf der Oberfläche eines Mikroarrays als besonders nachteilig.

Die Effizienz einer Hybridisierung an spezifischen Fängermolekülen lässt sich durch Anhäufung der Ziel-DNA-Einzelstränge gegenüber den jeweils komplementären Sequenzen signifikant steigern. Um diese Anhäufung einzelsträngiger DNA während einer PCR-Reaktion zu bewerkstelligen, kommen insbesondere zwei Verfahren zum Einsatz:
a) Asymmetrische PCR
b) Spezifische Entfernung eines DNA-Einzelstranges

Zu a) Bei der asymmetrischen PCR wird ein Primer (beispielsweise Sense-Primer) in wesentlich höherer Konzentration als der korrespondierende Primer (beispielsweise Antisense-Primer), die beide zur Erzeugung eines PCR-Produktes benötigt werden, dem Reaktionspuffer zugesetzt. Im Laufe einer PCR-Reaktion, d.h. nach einer gewissen Anzahl von Temperaturzyklen, liegen sowohl doppelsträngige als auch einzelsträngige DNA-Produkte - diese jedoch in wesentlich höherer Konzentration - vor. Es ist in diesem Fall keine Vereinzelung der Stränge durch temperaturinduzierte oder alkalische Denaturierung zwingend erforderlich. Ein Verfahren, bei dem ein erstes Primer-Paar und ein weiterer Zweit-Primer in unterschiedlichen Konzentrationen verwendet werden, ist beispielsweise in der Patentschrift DE 198 02 905 C2 offenbart.

Zu b) Bei der spezifischen Entfernung eines Einzelstranges wird in der Regel ein Primer mit einem Marker ausgestattet, beispielsweise einem Biotin-Molekül. Bei der nachfolgenden PCR-Reaktion ist ein Strang des doppelsträngigen DNA-Moleküls endständig biotinyliert. Biotin hat bekanntlich eine sehr hohe Affinität zu Streptavidin, mit dem es eine feste Bindung eingeht. Das Streptavidin befindet sich hierbei an einer Phase, wie beispielsweise magnetischen Beads. Nach einer Denaturierung der DNA-Doppel- in Einzelstränge kann in Folge der biotinylierte Einzelstrang von dem nicht markierten Einzelstrang mit Hilfe der Streptavidin gekoppelten magnetischen Beads herausgebunden und durch Anlagen eines magnetischen Felds entfernt werden. Alternativ können die biotinylierten Einzelstränge durch Überleitung über Streptavidin, das an eine feste Phase oder ein Resin gebunden vorliegt, abgetrennt werden. Dieses Verfahren ist bereits in dem Eastman Kodak Patent EP 0 418 960 A2 beschrieben.

Im Anschluss an die oben beschriebenen Verfahren können die Reaktionsprodukte direkt zur Hybridisierung an Fängermoleküle auf einem Mikroarray weiterverwendet werden. Beide Verfahren haben den Vorteil, dass gegenüber einer symmetrischen PCR keine Kompetition der fixierten Fängermoleküle mit frei in der Hybridisierungslösung vorliegenden einzelsträngigen, der Zielsequenz komplementären DNA-Stränge, auftreten können. Die Hybridisierung der Zielsequenzen mit den spezifischen Fängermolekülen ist somit weitaus effizienter als nach einer symmetrischen PCR-Reaktion und erfordert dadurch eine geringere Hybridisierungszeit. Die gesamte Assayzeit kann so durch Verkürzung der Hybridisierung maßgeblich beschleunigt werden. US2001/0018412 (Hitachi Ltd.) beschreibt PCR Verfahren mit an Beads gekoppelten Primern.

Die beschriebenen Verfahren weisen jedoch auch einige wesentlichen Nachteile auf:

Das grundlegende Problem bei der asymmetrischen PCR ist die Tatsache, dass sich die Bestimmung der Konzentration an forward and reverse Primer, um die optimale Ausbeute einzelsträngiger DNA zu erreichen, sehr aufwändig gestaltet. Die Parameter einer PCR-Reaktion, die abgesehen von der Konzentration von Magnesiumionen, freien Nukleotiden, Template-Konzentration und den freien Primern bestimmt wird, werden zusätzlich durch die Anzahl und Länge jedes einzelnen Temperaturzyklus und die Temperatur beeinflusst. Diese vielen unterschiedlichen Einflussgrößen müssen in langwierigen Experimenten optimiert werden, um eine für ein Mikroarray-Experiment erforderlichen Ausbeuten zu erreichen. Darüber hinaus gelten divergente Parameter jeweils für unterschiedliche Zielsequenzen und verwendete Primerpaare. Insbesondere beim Nachweis mehrerer unterschiedlicher Zielsequenzen, die durch eine einzige PCR-Reaktion erzeugt werden sollen (Multiplex - PCR), steigt die Komplexität eines derartigen Assays signifikant an. Ab einer Anzahl von etwa einem Dutzend verschiedener PCR-Produkte ist die Multiplex-Fähigkeit in der Regel erschöpft. Diese Limitation schränkt den Einsatz eines Mikroarrays zum Nachweis vieler biologischer Parameter trotz einer hohen Anzahl aufgebrachter Fängermoleküle maßgeblich ein.

Das beschriebene Problem erweist sich bei der zweiten Methode, der Biotinylierung eines Primers für die PCR und die nachfolgende Entfernung über die Bindung an Streptavidin, als weniger gravierend. Nachteile sind jedoch auch hier gegeben. Einerseits ist jedoch die Höhe des Multiplexens ebenfalls stark limitiert, andererseits sind zusätzliche Prozessschritte erforderlich, um die beiden Einzelstränge eines DNA-Doppelstrangs nachhaltig voneinander zu trennen. Dies setzt zusätzliche Anforderungen an die Mikrofluidik eines integrierten Systems.

Bei allen Verfahren tritt das Problem auf, dass die PCR-Produkte nicht rein vorliegen, sondern mit Primern, Nukleotiden, Enzymen etc. verunreinigt sind und daher in der Regel aufgereinigt werden müssen.

Es war daher die Aufgabe der vorliegenden Erfindung, ein Nachweisverfahren für Nukleinsäuren bereitzustellen, welches die oben beschriebenen Nachteile zumindest zum Teil überwindet.

Diese Aufgabe wird gelöst durch ein Verfahren gemäß Anspruch 1. Es handelt sich somit um ein Verfahren zum Nachweis mehrerer Zielnukleinsäuren, umfassend die Schritte
(i) Bereitstellen mehrerer Primer-Paare, jeweils bestehend aus einem ersten und einem zweiten Oligonukleotid-Primer, die geeignet sind, die Zielnukleinsäuren zu amplifizieren, wobei mehrere der ersten Primer an einen Semi-Festphasen-Träger gekoppelt sind und der jeweils zweite Primer frei vorliegt,
(ii) Bereitstellen einer Lösung, die eine nachzuweisende Zielnukleinsäure umfasst,
(iii) Inkontaktbringen der Primer-Paare aus (i) mit der Lösung aus (ii) und Durchführen einer Amplifikationsreaktion unter Bedingungen, bei denen die Zielnukleinsäure amplifiziert wird,
(iv) Denaturieren der doppelsträngigen Amplifikationsprodukte aus (iii) zu Einzelsträngen,
(v) Abtrennen des Semi-Festphasen-Trägers mit daran gekoppelten einzelsträngigen Amplifikationsprodukten und/oder mit daran gekoppelten unverlängerten Primern, und
(vi) Nachweisen der Zielnukleinsäuren.

Optional kann nach Schritt (iii) ein Waschschritt zur Entfernung der löslichen Komponenten des PCR-Reaktionsmixes (Nukleotide, Primer, Enzyme, Hilfsstoffe etc.) vorgesehen sein. Vorteilhafterweise können der erste und/oder der zweite Primer markiert sein.

Gemäß einer besonders bevorzugten Ausführungsform liegt der erste Primer nicht ausschließlich in an den Semi-Festphasen-Träger gekoppelter Form vor, sondern befindet sich auch frei in Lösung, z.B. in geringer Konzentration. Das Vorliegen des ersten Primers in freier, nicht gebundener Form wirkt sich günstig auf einen effizienten Start der Amplifikation aus.

Ein weiterer Gegenstand der vorliegenden Erfindung ist ein Kit zum Nachweis mehrerer Zielnukleinsäuren, umfassend
(a) mehrere Primer-Paare jeweils bestehend aus einem ersten und einem zweiten Oligonukleotid-Primer, die geeignet sind, die Zielnukleinsäure zu amplifizieren, wobei mehrere der ersten Primer an einen Semi-Festphasen-Träger gekoppelt sind und der jeweils zweite Primer frei vorliegt, und
(b) optional Reagenzien zur Durchführung einer Amplifikationsreaktion.

Bei der Zielnukleinsäure kann es sich um eine doppelsträngige Nukleinsäure handeln, z.B. DNA, cDNA, etc., aber auch einzelsträngige Nukleinsäuren, wie etwa RNA, wobei der komplementäre Strang gegebenenfalls vor der Reaktion ergänzt wird (z.B. durch Reverse Transkription).

Dieses erfindungsgemäße Verfahren eignet sich für komplexe Assay-Systeme, insbesondere für die PCR, insbesondere die Multiplex-PCR. Erfindungsgemäß wird vorgeschlagen, für ein solches Assay, das eine PCR-Reaktion oder Multiplex-PCR-Reaktion erfordert, einen Semi-Festphasen-Träger zu verwenden, auf dem sich jeweils spezifische Primer befinden. Als Semi-Festphasen-Träger ist grundsätzlich jedes granuläre Material geeignet, insbesondere sind Beads bevorzugt. Besonders bevorzugt handelt es sich um magnetische Beads. Solche Materialien sind bereits im Stand der Technik bekannt, z.B. epoxymodifizierte Magnetbeads (Dynal).

Die Erfindung wird nun anhand von Ausführungsbeispielen mit Bezug auf die beiliegenden Zeichnungen näher beschrieben.

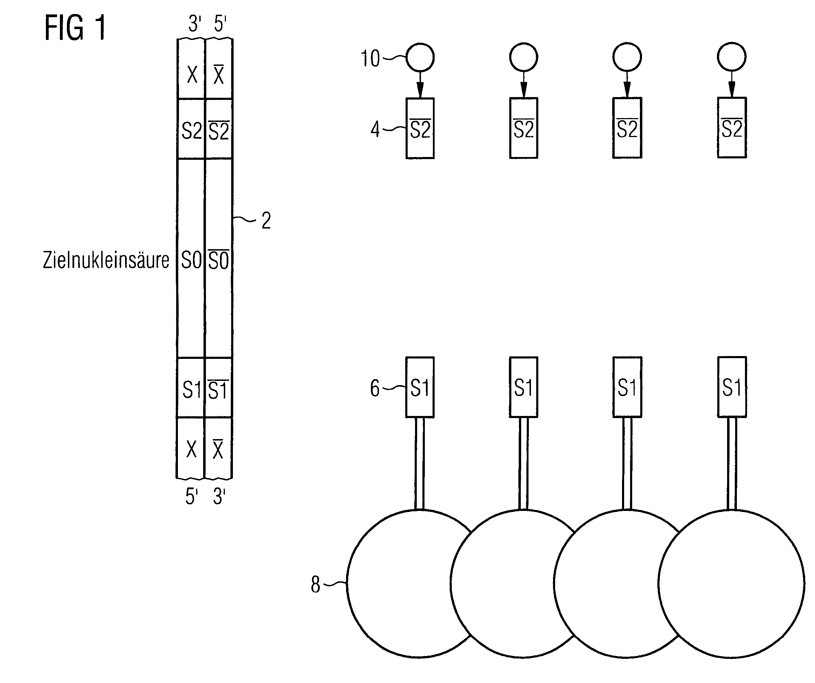

Fig. 1 zeigt eine schematische Darstellung der Zielsequenz 2 und der ersten Primer 4, die mit einem Marker 10 versehen sind, sowie der zweiten Primer 6, die an ein magnetische Bead 8 gekoppelt sind.

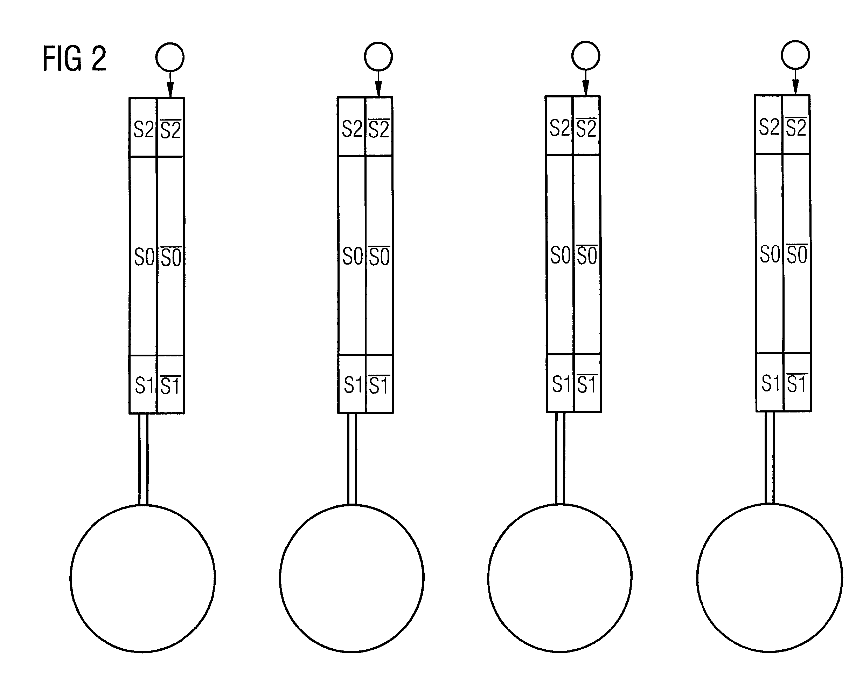

Fig. 2 zeigt schematisch die doppelsträngigen Bead-gebundenen Amplifikationsprodukte nach der Amplifikation.
Fig. 3 zeigt schematisch die einzelsträngigen Amplifikationsprodukte nach der Denaturierung der doppelsträngigen Amplifikationsprodukte.
Fig. 4 zeigt schematisch die magnetische Trennung der Bead-gebundenen einzelsträngigen Amplifikationsprodukte von den markierten doppelsträngigen Amplifikationsprodukten.
Fig. 5 zeigt schematisch die Hybridisierung der markierten einzelsträngigen Amplifikationsprodukte mit komplementären Fängermolekülen 22 und deren Nachweis, der z.B. optisch oder elektrisch oder auf andere Art und Weise durchgeführt werden kann.
Fig. 6 zeigt schematisch eine alternative Hybridisierung der Bead-gebundenen einzelsträngigen Amplifikationsprodukte mit immobilisierten komplementären Fängermolekülen 22 und deren magnetischen Nachweis.

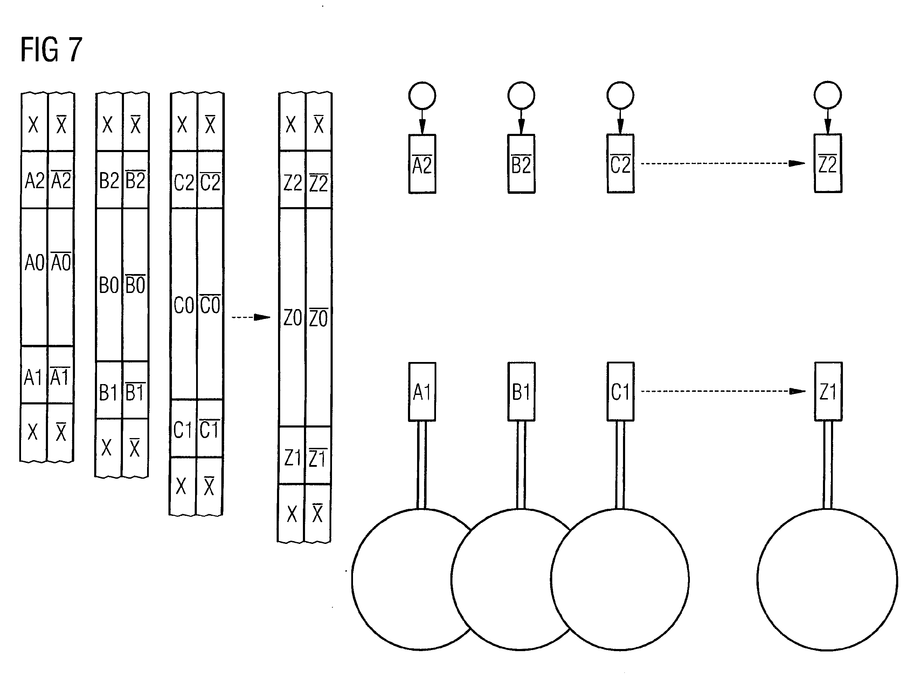

Fig. 7 zeigt schematisch eine Multiplex-PCR-Reaktion mit unterschiedlichen markierten freien und Bead-gebundenen Primern, die gegen verschiedene Zielsequenzen gerichtet sind.

Jeder Träger-Partikel, z.B. jedes individuelle magnetische Bead ist mit mindestens einem spezifischen Typen von Oligonukleotid-Primer ausgestattet. Die korrespondierenden zweiten Primer für die spezifischen PCR-Produkte liegen hingegen frei in Lösung vor. Ein Teil der PCR-Reaktion findet somit an einer Art semi-festen Phase (dem magnetischen Bead) statt. Da die Beads während der Reaktion bewegt werden können, um die Diffusion zu erleichtern, kann die PCR definitionsgemäß als Semi-Festphasen-PCR bezeichnet werden. Bei einer PCR-Multiplex-Reaktion unter Einsatz der Semifestphasen Technologie können zwei Varianten gewählt werden:

Erste Variante: Jeweils ein Primer des eine spezifische Sequenz amplifizierenden Primer-Paares befindet sich auf einem Typen eines magnetischen Beads (Sense oder Antisense). Der korrespondierende Primer (Antisense oder Sense) befindet sich frei in der Reaktionslösung. Auf einem Bead befindet sich also nur jeweils ein spezifischer Primer. Bei einer Multiplex-PCR werden für X PCR-Produkte X verschieden funktionalisierte magnetische Beads verwendet. Die X korrespondierenden Primer befinden sich frei in der Reaktionslösung.

Zweite Variante: Mehr als ein oder alle für eine Multiplex-PCR benötigten (Sense oder Antisense) Primer eines Primer-Paares befinden sich auf einem Typen eines magnetischen Beads. Die jeweils korrespondierenden Primer für das jeweilige PCR-Produkt befinden sich frei in der Reaktionslösung. Somit tragen alle verwendeten Beads mehr als eine Funktion (Multivalenz) auf der Oberfläche. Bei einer PCR-Multiplex mit X verschiedenen PCR-Produkten kommen X Primer-Paare zum Einsatz. Insgesamt X verschiedene Primer für eine Seite (Sense oder Antisense) einer zu amplifizierenden DNA-Sequenz befinden sich alle auf einem Typ eines magnetischen Beads. Die jeweils korrespondierenden X Primer eines jeden Primer-Paares liegen wiederum frei in Lösung vor.

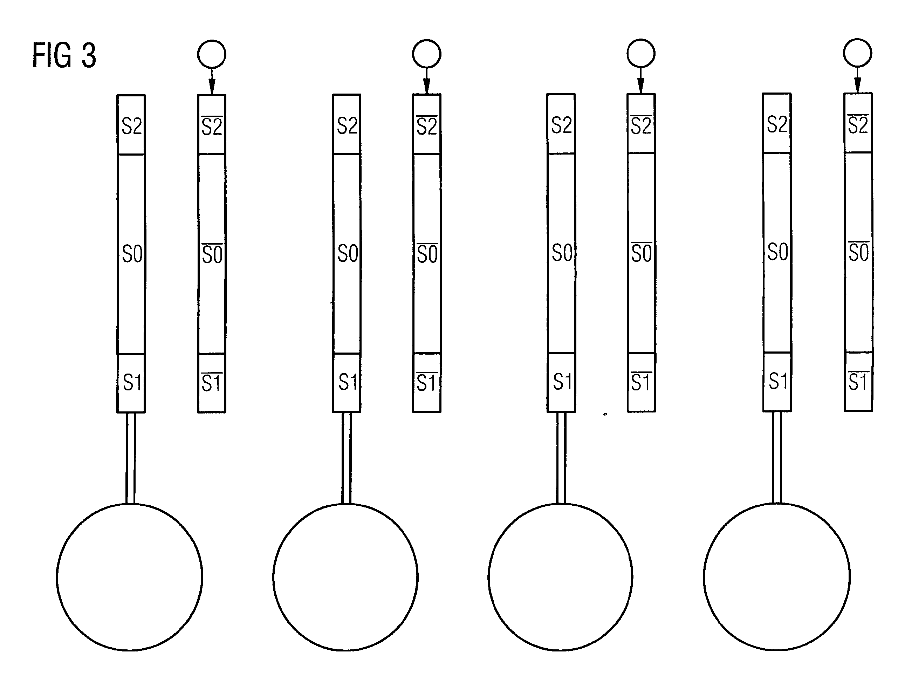

Beim Fortgang der PCR-Reaktion wird jeweils ein DNA-Strang am Bead, der andere Strang eines PCR-Produkts am freien Primer elongiert. Beim Abschluss der PCR-Reaktion liegt ein Großteil der Primer am Bead in doppelsträngiger verlängerter Form vor. Führt man nun die Denaturierung der Doppelstränge durch, so befindet sich ein Einzelstrang eines jeden PCR-Produktes frei in Lösung, der jeweils komplementäre Einzelstrang am Bead gebunden vor. Dies ist schematisch z.B. in Fig. 3 dargestellt.

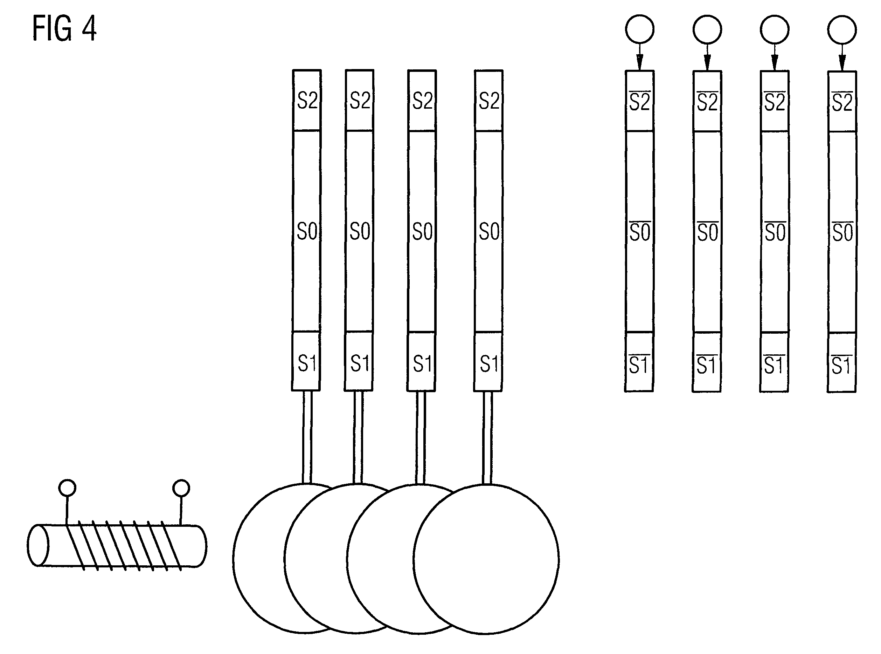

Bei einer folgenden Hybridisierungsreaktion auf einem Mikroarray hat man jetzt die Möglichkeit, sämtliche magnetischen Beads mittels eines Magneten in der PCR-Kammer zurückzuhalten und die frei in Lösung befindlichen einzelsträngigen PCR-Produkte in die Hybridisierungskammer zu befördern. Die noch in Lösung befindlichen einzelsträngigen PCR-Produkte hybridisieren jetzt ausschließlich mit den komplementären auf dem Mikroarray befindlichen Fängermolekülen, da die am Bead befindlichen komplementären Einzelstränge nicht mehr erreichbar sind. Auf diese Weise wird es möglich, ein der asymmetrischen PCR äquivalentes Verfahren zu realisieren, ohne eine komplexe Titrierung der korrespondieren Primer durchführen zu müssen. Die Abtrennung ist schematisch in Fig. 4 dargestellt.

Die Primer können auch zusätzlich markiert sein. Hierzu eigenen sich im Stand der Technik bekannte Nukleinsäure-Marker, wie z.B. Biotin, Fluoreszenzmarker u. dgl. Es ist besonders vorteilhaft, wenn der zweite Primer, d.h. der nicht an den Träger gekoppelte Primer, markiert ist. Es können aber auch beide Primer eines Primer-Paares markiert sein.

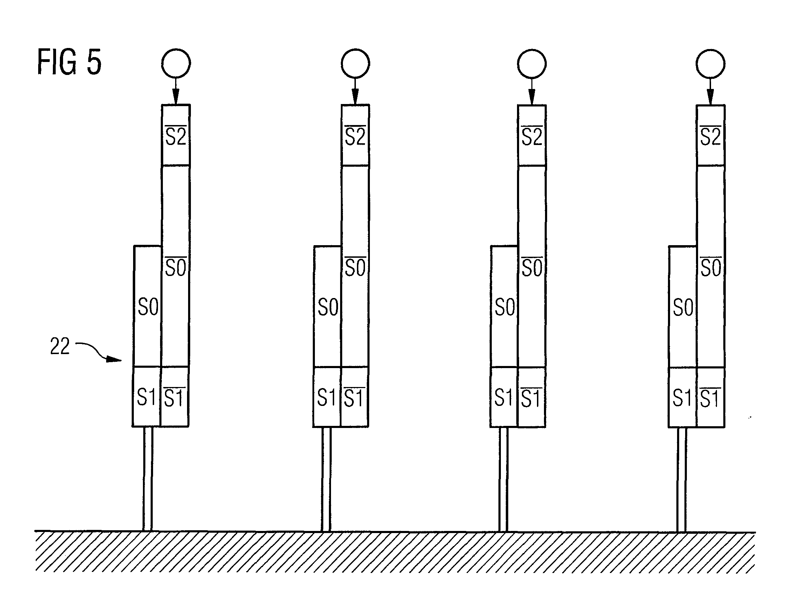

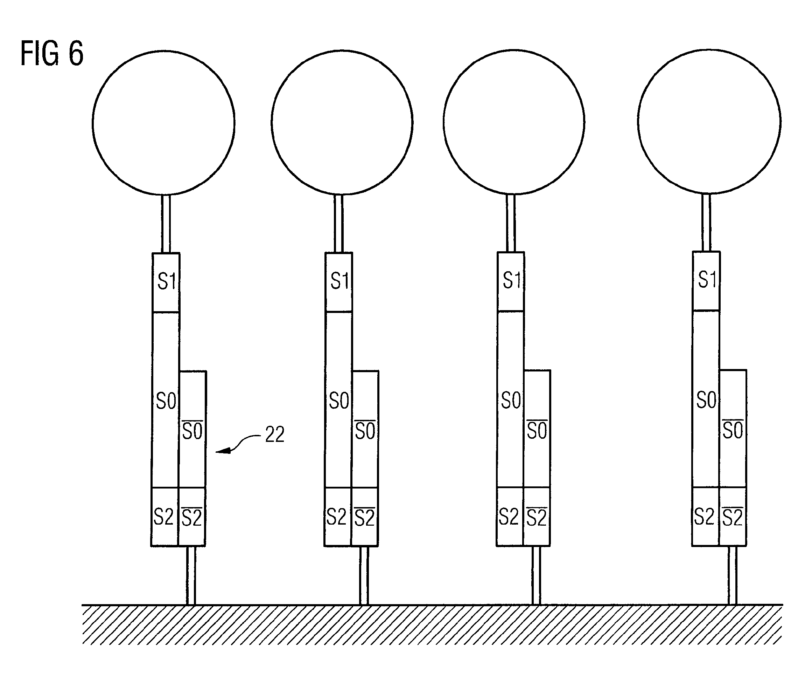

Im Anschluss an die Abtrennung wird die amplifizierte Zielnukleinsäure nachgewiesen. Dies kann auf mehrere Arten geschehen. Eine Möglichkeit ist der Nachweis durch Hybridisierung mit spezifischen Fängermolekülen, welche z.B. markiert oder auf einem festen Träger immobilisiert sein können. Es können hierbei entweder die mit dem ersten Primer oder die mit dem zweiten Primer amplifizierten Stränge nachgewiesen werden. Bei einer Variante sind diese Einzelstränge markiert, bei einer zweiten Variante handelt es sich um die an die Beads gekoppelten Primer-elongierten Einzelstränge. Diese Varianten sind z.B. in Fig. 5 und 6 dargestellt.

Eine sinnvolle Abwandlung der Erfindung stellt die Amplifizierung (PCR), Hybridisierung auf dem Mikroarray und der Nachweis der Hybridisierungsereignisse in einer Kammer dar. Im Falle einer einzigen Kammer werden nach Abschluss der PCR-Reaktion die magnetischen Beads nach der Denaturierung an die entgegengesetzte Seite des Mikroarrays oder alternativ aus der Kammer durch ein von außen angelegtes Magnetfeld befördert. Auf diese Weise reagieren die Einzelstränge bevorzugt bzw. ausschließlich mit den komplementären Fängermolekülen auf dem Mikroarray.

Der grundlegende Vorteil der Verwendung magnetischer Beads in einer PCR-Multiplex Reaktion liegt in der einfachen Realisierbarkeit einzelsträngiger gereinigter PCR-Produkte für nachfolgende Hybridisierungsreaktionen. Auf diese Weise kann auf die aufwändige Optimierung asymmetrischer PCR-Reaktionen verzichtet werden. Im Vergleich zu symmetrischen PCR-Reaktionen ist die Ausbeute einzelsträngiger PCR-Produkte wesentlich erhöht, was zu einer höheren Effizienz bei nachfolgenden Mikroarray-Experimenten und damit höherer Empfindlichkeit des Assays führt.

Dieser Vorteil kommt ganz besonders beim PCR-Multiplexing zu tragen, da hier der Erzeugung mehrerer PCR-Produkte in einer Reaktion enge Grenzen gesetzt sind. Sowohl bei der symmetrischen als auch der asymmetrischen Multiplex-PCR schwanken die Ausbeuten zwischen den verschiedenen PCR-Produkten oft beträchtlich. Diese Schwankung wird bei der Verwendung magnetischer Bead-gekoppelter Primer dadurch geglättet, dass die Amplifikation einer mehr linearen als exponentiellen Reaktionskinetik folgt, als dies bei der Verwendung freier Primer der Fall ist.

Die Semi-Festphasen-Reaktion unter Einsatz von magnetischen Beads hingegen adressiert einen Grossteil der Unzulänglichkeiten einer konventionellen symmetrischen und asymmetrischen PCR-Multiplex Reaktion:
Die aufwändige Optimierung der Parameter, die bei Multiplex-Reaktionen, insbesondere asymmetrischen PCR-Multiplex-Reaktionen nicht erforderlich ist.
Die Assaygeschwindigkeit, die insbesondere aufgrund der verkürzten Hybridisierungsdauer beschleunigt wird.
Die Ausbeuten einzelsträngiger PCR-Produkte ist wesentlich höher als bei einer vergleichbaren asymmetrischen PCR, da sämtliche komplementären DNA-Stränge eines jeweiligen PCR-Produktes auf der Bead-Oberfläche gebunden vorliegen und bei nachfolgenden Hybridisierungsreaktionen nicht stören.
Ein wesentlich höherer Multiplexgrad im Gegensatz zu konventionellen homogenen PCR-Reaktionen aufgrund der günstigeren Reaktionskinetik wird möglich.
Die am Bead elongierten einzelsträngigen PCR-Produkte zusätzlich durch magnetoresistive Detektionstechnologien (beispielsweise GMR- oder TMR-Sensoren) können nachgewiesen werden. Diese Möglichkeit eröffnet den Einsatz alternativer und womöglich vorteilhafter online Detektionsverfahren.

## Patentansprüche

1. Verfahren zum Nachweis mehrerer verschiedener Zielnukleinsäuren, umfassend die Schritte
(i) Bereitstellen mehrerer verschiedener Primer-Paare, jeweils bestehend aus einem ersten und einem zweiten Oligonukleotid-Primer, die geeignet sind, die Zielnukleinsäuren zu amplifizieren, wobei mehrere der ersten Primer an einen magnetischen Bead gekoppelt sind und der jeweils zweite Primer frei vorliegt,
(ii) Bereitstellen einer Lösung, die nachzuweisende Zielnukleinsäuren umfasst,
(iii) Inkontaktbringen der Primer-Paare aus (i) mit der Lösung aus (ii) und Durchführen einer Amplifikationsreaktion unter Bedingungen, bei denen die Zielnukleinsäuren amplifiziert werden,
(iv) Denaturieren der doppelsträngigen Amplifikationsprodukte aus (iii) zu Einzelsträngen,
(v) Abtrennen der magnetischen Beads mit daran gekoppelten einzelsträngigen Amplifikationsprodukten und/oder mit daran gekoppelten unverlängerten Primern, und
(vi) Nachweisen der Zielnukleinsäuren.

2. Verfahren nach einem der vorhergehenden Ansprüche, wobei nach Schritt (iii) ein waschschritt zur Entfernung der Primer und ggf. anderer löslicher Komponenten der Amplifikations-Reaktionslösung vorgesehen ist.

3. Verfahren nach einem der vorhergehenden Ansprüche, wobei die ersten Primer nicht ausschließlich in an den magnetischen Bead gekoppelter Form vorliegen, sondern sich auch frei in Lösung befinden.

4. Verfahren nach einem der vorhergehenden Ansprüche, wobei mindestens einer der ersten und/oder der zweiten Primer markiert ist.

5. Verfahren nach einem der vorhergehenden Ansprüche, wobei der jeweils zweite Primer markiert ist.

6. Verfahren nach einem der vorhergehenden Ansprüche, wobei der Nachweis mit Hilfe von spezifischen Fängermolekülen erfolgt.

7. Kit zum Nachweis mehrerer verschiedener Zielnukleinsäuren, umfassend
(a) mehrere verschiedene Primer-Paare jeweils bestehend aus einem ersten und einem zweiten Oligonukleotid-Primer, die geeignet sind, die Zielnukleinsäuren zu amplifizieren, wobei mehrere der ersten Primer an einen magnetischen Bead gekoppelt sind und der jeweils zweite Primer frei vorliegt, und
(b) optional Reagenzien zur Durchführung einer Amplifikationsreaktion.

8. Kit nach Anspruch 7, wobei mindestens einer der ersten und/oder der zweiten Primer markiert ist.

9. Kit nach Anspruch 8, wobei der jeweils zweite Primer markiert ist.

10. Kit nach einem der vorhergehenden Ansprüche 7 bis 9, zusätzlich umfassend spezifische Fängermoleküle.

## Claims

1. Process for detecting a plurality of different target nucleic acids, comprising the following steps:
(i) providing a plurality of different primer pairs consisting of in each case a first and a second oligonucleotide primer suitable for amplifying said target nucleic acids, with a plurality of said first primers being coupled to a magnetic bead and the in each case second primer being free,
(ii) providing a solution comprising target nucleic acids to be detected,
(iii) contacting the primer pairs of (i) with the solution of (ii) and conducting an amplification reaction under conditions in which the target nucleic acids are amplified,
(iv) denaturing the double-stranded amplification products of (iii) to give single strands,
(v) removing the magnetic beads with single-stranded amplification products coupled thereto and/or with non-extended primers coupled thereto, and
(vi) detecting the target nucleic acids.

2. Process according to the preceding claim, wherein step (iii) is followed by a washing step to remove the primers and, where appropriate, other soluble components of the amplification reaction solution.

3. Process according to either of the preceding claims, wherein the first primers are present not only coupled to the magnetic bead but also free in solution.

4. Process according to any of the preceding claims, wherein at least one of the first and/or the second primers is labeled.

5. Process according to any of the preceding claims, wherein the in each case second primer is labeled.

6. Process according to any of the preceding claims, wherein detection is carried out with the aid of specific capture molecules.

7. Kit for detecting a plurality of different target nucleic acids, comprising
(a) a plurality of different primer pairs consisting of in each case a first and a second oligonucleotide primer suitable for amplifying said target nucleic acids, with a plurality of said first primers being coupled to a magnetic bead and the in each case second primer being free, and
(b) optionally reagents for carrying out an amplification reaction.

8. Kit according to Claim 7, wherein at least one of the first and/or the second primers is labeled.

9. Kit according to Claim 8, wherein the in each case second primer is labeled.

10. Kit according to any of the preceding Claims 7 to 9, additionally comprising specific capture molecules.

## Revendications

1. Procédé de détection de plusieurs acides nucléiques cibles différents, comprenant les stades
(i) on se procure plusieurs paires d'amorces différentes constituées respectivement d'une première et d'une deuxième amorces oligonucléotide, qui sont propres à amplifier les acides nucléiques cibles, plusieurs des premières amorces étant couplées à une perle magnétique et respectivement la deuxième amorce étant libre,
(ii) on se procure une solution qui comprend des acides nucléiques cibles à détecter,
(iii) on met les paires d'amorce de (i) en contact avec la solution de (ii) et on effectue une réaction d'amplification dans des conditions dans lesquelles les acides nucléiques cibles sont amplifiés,
(iv) on dénature les produits d'amplification à double brin de (iii) en des monobrins,
(v) on sépare les perles magnétiques avec les produits d'amplification monobrins qui y sont couplés et/ou avec les amorces non prolongées qui y sont couplées, et
(vi) on détecte les acides nucléiques cibles.

2. Procédé suivant l'une des revendications précédentes, dans lequel après le stade (iii) on prévoit un stade de lavage pour éliminer les amorces et le cas échéant d'autres constituants solubles de la solution de réaction d'amplification.

3. Procédé suivant l'une des revendications précédentes, dans lequel les premières amorces ne se présentent pas exclusivement sous la forme couplée à la perle magnétique, mais se trouvent aussi librement en solution.

4. Procédé suivant l'une des revendications précédentes, dans lequel au moins l'une des premières et/ou des deuxièmes amorces est marquée.

5. Procédé suivant l'une des revendications précédentes, dans lequel respectivement la deuxième amorce est marquée.

6. Procédé suivant l'une des revendications précédentes, dans lequel on effectue la détection à l'aide de molécules de capture spécifiques.

7. Trousse de détection de plusieurs acides nucléiques de cibles différentes, comprenant
(a) plusieurs paires d'amorces différentes constituées respectivement d'une première et d'une deuxième amorce oligonucléotide, qui sont propres à amplifier les acides nucléiques cibles, plusieurs des premières amorces étant couplées à une perle magnétique et respectivement la deuxième amorce étant libre,
(b) des réactifs facultatifs pour effectuer une réaction d'amplification.

8. Trousse suivant la revendication 7, dans laquelle au moins l'une des premières et/ou des deuxièmes amorces est marquée.

9. Trousse suivant la revendication 8, dans laquelle respectivement la deuxième amorce est marquée.

10. Trousse suivant l'une des revendications précédentes 7 à 9, comprenant en outre des molécules de capture spécifiques.
